# FASCICULE DE BREVET EUROPEEN

(11) **EP 0 816 324 B1**
(45) Date de publication et mention de la délivrance du brevet: **16.02.2000**
(21) Numéro de dépôt: 97401397.1
(22) Date de dépôt: 18.06.1997
(51) Int. Cl.: C07C 217/54, A61K 31/135, A61K 7/48

(54) **Dérivés benzyle (s) substitué (s) de polyalkyléne-polyamines et leur utilisation dans des compositions cosmétiques et pharmaceutiques**
Substituierte Benzylderivate von Polyalkylenpolyaminen und ihre Verwendung in kosmetischen und pharmazeutischen Zusammensetzungen
Subsituted benzyl derivatives of polyalkylene-polyamines and their use in cosmetic and pharmaceutical compositions

(30) Priorité: 18.06.1996 FR 9607541
(43) Date de publication de la demande: 07.01.1998
(73) Titulaire: L'OREAL, 75008 Paris (FR)
(72) Inventeur: Galey, Jean-Baptiste, 93600 Aulnay-Sous-Bois (FR)
(74) Mandataire: Stalla-Bourdillon, Bernard

(56) Documents cités:
- EP-A- 0 277 635
- WO-A-94/07480
- WO-A-94/11338
- JOURNAL OF ORGANOMETALLIC CHEMISTRY, vol. 490, 22 Mars 1995, LAUSANNE CH, pages 143-148, XP000645089 PAUL D. BEER ET AL.: "Towards conformationally dependent redox-responsive molecular switches...."

## Description

La présente invention a pour objet des nouveaux dérivés benzyle(s) (s) substitué(s) (s) de polyalkylène-polyamines qui trouvent une utilisation dans des compositions cosmétiques et pharmaceutiques en vue de protéger l'organisme contre le stress oxydant.

Le stress oxydant est la résultante d'un certain nombre de situations physiologiques et physiopathologiques qui entrainent un déséquilibre de la balance antioxydant prooxydant.

Ce déséquilibre se traduit essentiellement par des processus oxydatifs non contrôlés au sein des tissus vivants mettant en jeu des radicaux libres oxygénés et conduisant à la formation de dégâts oxydatifs sur les molécules et macromolécules biologiques.

Un certain nombre de situations physiopathologiques provoquent, favorisent, accompagnent ou sont la conséquence directe d'un stress oxydant. Il s'agit notamment de l'inflammation, du vieillissement, de l'exposition aux ultraviolets, de la carcinogenèse, de la toxicité et/ou du mode d'action de certains médicaments.

On sait qu'au cours d'un stress oxydant, du fer est libéré de ses sites de stockage normaux comme la ferritine et devient alors accessible pour participer à certaines réactions, et notamment aux réactions de Fenton et Haber-Weiss, permettant ainsi la formation de radicaux hydroxyles, ceux-ci étant connus pour être responsables de nombreux dommages oxydatifs.

On a, depuis longtemps, recherché divers composés permettant de protéger l'organisme contre le stress oxydant et, en particulier, des composés de synthèse.

Ces composés peuvent être regroupés dans les principales classes suivantes :
- les antilipoperoxydants, tels que la vitamine E, le trolox, le butylhydroxytoluène,
- les réducteurs biologiques, comme le glutathion réduit et ses dérivés, la vitamine C et ses dérivés,
- les désactivateurs d'oxygène singulet (quenchers), comme le β-carotène,
- les systèmes capables de décomposer le peroxyde d'hydrogène et, en particulier, des enzymes telles la catalase ou des peroxydases en présence de leurs co-substrats,
- les systèmes de protection contre l'anion superoxyde, comme la superoxyde dismutase (SOD), le complexe Mn-desferal ou le di-isopropyl salicylate de cuivre,
- les systèmes capables de décomposer les hydroperoxydes organiques comme la glutathion peroxydase ou des systèmes modèles à base de sélénium,
- les chélateurs du fer comme le desferal ou certaines hydroxypyridinones.

Toutefois, il s'est avéré qu'aucun de ces composés n'était réellement efficace en vue de protéger l'organisme vis-à-vis des radicaux hydroxyles. Si, toutefois, certains d'entre eux ont montré une certaine efficacité en tant que piégeur de radicaux hydroxyles, il s'est avéré que ceux-ci ne l'étaient qu'à des concentrations élevées, ce qui les rendaient totalement inutilisables *in vivo*.

Il a toutefois été proposé plus récemment dans la demande de brevet WO-94/11338 certains composés susceptibles de former des complexes avec le fer dont les constantes de stabilité sont faibles, ce qui diminue, par conséquent, les risques de toxicité associés à leur utilisation.

A partir de cet état de la technique, de nouveaux composés ont été synthétisés et il a été constaté que ceux-ci exerçaient une protection particulièrement efficace contre l'attaque des radicaux hydroxyles générés par les réactions de Fenton et Haber-Weiss.

Les nouveaux composés selon l'invention, tels qu'ils seront définis ci-après, trouvent une utilisation comme substances actives pour se protéger des effets néfastes des radicaux libres, c'est-à-dire contre le stress oxydant et notamment pour traiter des situations pathologiques en médecine humaine ou vétérinaire comme les cancers, les états inflammatoires, l'ischémie reperfusion, les surcharges en fer, les maladies dégénératives du système nerveux, ou encore pour traiter les effets de l'utilisation de certains médicaments connus pour générer des radicaux libres et, notamment, des anticancéreux comme l'adriamycine.

Les composés selon l'invention trouvent également une utilisation dans des situations non pathologiques, comme l'exposition au soleil ou le vieillissement pour protéger notamment la peau ou les cheveux.

La présente invention a donc pour objet des dérivés benzyle(s) substitué(s) de polyalkylène-polyamines répondant à la formule : 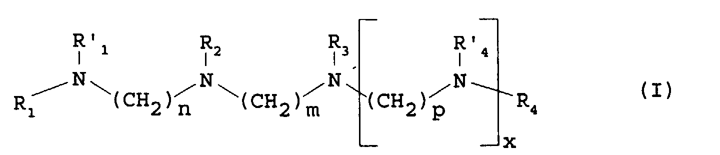 dans laquelle :
n et m = 2, 3 ou 4,
p = 2 ou 3,
x = 0, 1 ou 2,
R₁ et R₄, identiques ou différents, représentent
(i) un radical alkyle, linéaire ou ramifié, en C₁-C₄, ou
(ii) un radical de formule : 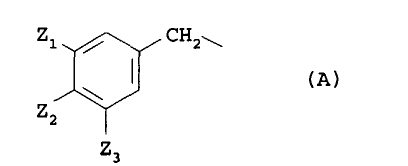
dans laquelle :
Z₁ représente OH ou OR,
Z₂ et Z₃ représentent H, OH ou OR, R étant un radical alkyle, linéaire ou ramifié, en C₁-C₈,
R₂ et R₃, identiques ou différents, représentent H, un radical alkyle, linéaire ou ramifié, en C₁-C₄, un radical de formule (A) ou un radical de formule (B) suivante : 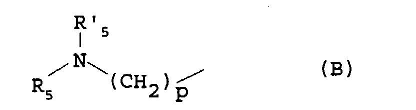
R'₁, R'₄ et R'₅ représentent H ou un radical alkyle, linéaire ou ramifié, en C₁-C₄, et
R₅ représente un radical alkyle, linéaire ou ramifié, en C₁-C₄ ou un radical de formule (A),
sous réserve que l'un au moins des radicaux R₁, R₂, R₃, R₄ ou R₅ représente un radical de formule (A), et que lorsque R₁ et R₄, identiques, représentent un radical de formule (A), x étant 0 ou 1, Z₁ ou Z₁ et Z₂, identiques, ne peuvent représenter OR, R étant un radical alkyle en C₁-C₆,
et leurs sels et complexes métalliques.

Par radical alkyle, linéaire ou ramifié en C₁-C₄, on doit entendre des radicaux tels que méthyle, éthyle, isopropyle et tert-butyle.

Lorsque le radical alkyle, linéaire ou ramifié, comporte jusqu'à 8 atomes de carbone, celui-ci peut être alors en outre le radical pentyle, hexyle, isohexyle ou octyle.

Selon une première forme particulièrement préférée des composés selon l'invention, l'un au moins des radicaux R₁, R₂, R₃, R₄ et/ou R₅ représente un radical de formule (A) dans laquelle Z₁, Z₂ et Z₃, identiques ou différents, représentent OH ou OR, R étant un alkyle, linéaire ou ramifié, en C₁-C₈.

Enfin, selon une deuxième forme tout particulièrement préférée des composés selon l'invention, les radicaux Z₁, Z₂ et Z₃ sont identiques et représentent un groupe méthoxy, le radical de formule (A) étant le radical 3,4,5-triméthoxybenzyle.

Parmi les sels des composés de formule (I), on peut notamment citer les sels d'addition d'un acide minéral tel que l'acide sulfurique, l'acide chlorhydrique, l'acide nitrique ou l'acide phosphorique.

Parmi les complexes, on peut citer ceux formés par addition du chlorure de zinc ou du chlorure de calcium.

A titre d'exemple, on peut citer comme composés représentatifs des composés de formule (I), les suivants :
N-(3,4,5-triméthoxybenzyl)-N'{2-[2-(3,4,5-triméthoxybenzylamino)-éthylamino]-éthyl}-éthane-1,2-diamine (tétrachlorhydrate),
N-(3,4,5-triméthoxybenzyl)-N'(2-{2-[2-(3,4,5-triméthoxybenzylamino)-éthylamino]-éthylamino}-éthyl)-éthane-1,2-diamine (pentachlorhydrate),
N,N'-bis-[3-(3,4,5-triméthoxybenzylamino)-propyl]-butane-1,4-diamine-(tétrachlorhydrate),
N,N'-bis-(2-diéthylaminoéthyl)-N,N'-bis-(3,4,5-triméthoxybenzyl)-éthane-1,2-diamine,
N-(3,4,5-triméthoxybenzyl)-N',N'-bis[2-(3,4,5-triméthoxybenzylamino)-éthyl]éthane-1,2-diamine.

Le procédé de préparation des composés selon l'invention est réalisé selon les modes de synthèse conventionnels qui consistent à faire réagir une polyalkylène-polyamine avec un benzaldéhyde mono- ou poly-substitué en milieu solvant organique à une température inférieure au point d'ébullition du solvant. La mono- ou polybenzylidène amine obtenue (base de Schiff), isolée ou non, est ensuite réduite en présence de borohydrure de sodium ou par hydrogénation catalytique. Lorsque les composés selon l'invention présentent un radical de formule (B), on fait alors réagir sur le produit résultant de l'hydrogénation, une halogénoalkyl mono- ou dialkylamine dans les conditions classiques pour ce type de réaction.

Parmi les polyalkylène-polyamines de départ, on peut notamment citer l'éthylène diamine, la diéthylène triamine, la trientine (ou triéthylène tétramine), la tétraéthylène pentamine, la spermine (ou N,N'-bis(3-aminopropyl)-1,4 butane diamine) et la tris(2-aminoéthyl)amine.

Parmi les benzaldéhydes substitués, on peut notamment citer le 3,4,5-triméthoxy-benzaldéhyde, le 3,5-diméthoxy-benzaldéhyde, le 3-hydroxy-benzaldéhyde, le 3,5-dihydroxy-benzaldéhyde, le 3-hydroxy-4-éthoxy-benzaldéhyde, etc...

Parmi les halogénoalkyl mono- ou dialkylamines, on peut notamment citer la bromoéthyl diéthylamine, la bromoéthyl diméthylamine, etc...

Les exemples donnés ci-après permettent d'illustrer les méthodes d'accès aux composés selon l'invention.

La présente invention a en outre pour objet une composition cosmétique ou pharmaceutique contenant au moins un composé de formule (I) ou l'un de ses sels ou complexes métalliques dans un véhicule cosmétiquement ou pharmaceutiquement acceptable.

Dans ces compositions, le composé actif de formule (I) est généralement présent en une proportion de 0,001 à 10 % en poids par rapport au poids total de la composition.

Les compositions cosmétiques peuvent se présenter sous diverses formes conventionnelles telles que sous forme d'onguent, de crème, de pommade, de gel, de spray, de lotion, d'émulsion ou de dispersion vésiculaire.

Dans les compositions selon l'invention, il a été constaté par ailleurs que les composés de formule (I) jouaient un rôle important par leur action antioxydante et permettaient ainsi de les protéger de l'oxydation.

Lorsque le composé actif de formule (I) est utilisé dans le cadre d'un traitement pharmaceutique, les formes d'administration peuvent être par voie orale, topique ou parentérale, le support pharmaceutiquement acceptable étant fonction de la forme d'administration choisie.

Les doses d'administration sont généralement comprises entre 1 et 1000 mg/Kg/jour.

Les compositions pharmaceutiques selon l'invention sont tout particulièrement destinées à traiter des situations de stress oxydant liées à certains états pathologiques et, notamment, les maladies neuro-dégénératives telles que par exemple la maladie de Parkinson, les situations inflammatoires chroniques, le syndrome d'ischémie reperfusion, la toxicité de certains médicaments tels que certains xénobiotiques et les surcharges en fer.

Selon une forme de réalisation préférée des compositions selon l'invention, le composé de formule (I) peut être associé à au moins une autre substance active (ou une autre substance anti-radicaux libres). Ces substances peuvent être choisies plus particulièrement parmi celles énumérées précédemment de la page 1, ligne 29 à la page 2, ligne 5.

Les composés actifs de formule (I) et les substances actives ou les substances anti-radicaux libres peuvent être associés au sein de la même composition ou être appliqués séparément.

Les exemples ci-après sont donnés en vue d'illustrer la préparation des composés de formule (I) et leurs utilisations dans les domaines pharmaceutiques et cosmétiques.

### Exemple 1 : Préparation du tétrachlorhydrate de N-(3,4,5-triméthoxy-benzyl)-N'{2-[2-(3,4,5-triméthoxybenzylamino)-éthylamino]-éthyl}-éthane-1,2-diamine

A 5 g de triéthylène tétramine solubilisés dans 200 ml de toluène, on ajoute 13,40 g de 3,4,5-triméthoxybenzaldéhyde. Après installation d'un Dean Stark, le milieu réactionnel est porté au reflux pendant 6h et on évapore à sec.

Le résidu obtenu est repris dans 200 ml d'éthanol auquel on ajoute 2,60 g de borohydrure de sodium à température ambiante. Après 24h d'agitation et addition de 200 ml d'eau, le milieu est concentré sous vide de moitié. La solution résiduelle est amenée à pH 10 par addition de soude concentrée puis extraite par 4 x 100 ml de dichlorométhane. La phase organique est lavée par une solution à 10 % NaCl puis séchée sur sulfate de sodium. Après évaporation du solvant, on obtient une huile jaune (14,70 g) qui est reprise dans 70 ml de méthanol chlorhydrique 2N. Après 1h d'agitation, le précipité formé est filtré sur verre fritté et lavé par 2 x 50 ml d'éther éthylique. On le recristallise dans 250 ml d'un mélange éthanol-eau 9/1 et obtient 6,80 g de tétrachlorhydrate de N-(3,4,5-triméthoxybenzyl)-N'{2-[2-(3,4,5-triméthoxybenzylamino)-éthylamino]-éthyl}-éthane-1,2-diamine sous forme d'une poudre blanche.

Point de fusion = 226°C
Spectre RMN (500 MHz) dans DMSO : conforme à la structure attendue.
Analyse élémentaire : Produit sous forme de tétrachlorhydrate, monohydrate

| | C | H | N | O | Cl |
|---|---|---|---|---|---|
| Calc. % | 46,57 | 7,16 | 8,35 | 16,71 | 21,19 |
| Tr. % | 46,59 | 7,19 | 8,15 | 16,97 | 21,03 |

### Exemple 2 : Préparation du pentachlorhydrate de N-(3,4,5-triméthoxy-benzyl)-N'(2-{2-[2-(3,4,5-triméthoxybenzylamino)-éthylamino]-éthylamino}-éthyl)-éthane-1,2-diamine

A partir de 5 g de tétraéthylène pentamine et selon les mêmes conditions opératoires que celles décrites à l'exemple 1, on obtient 3,1 g de pentachlorhydrate de N-(3,4,5-triméthoxybenzyl)-N'(2-{2-[2-(3,4,5-triméthoxybenzylamino)-éthylamino]-éthylamino}-éthyl)-éthane-1,2-diamine sous forme d'une poudre blanche.

Point de fusion : 220°C (dec.) (Kofler)
Spectre RMN (500 MHz) dans DMSO : conforme à la structure attendue.
Analyse élémentaire : Produit sous forme pentachlorhydrate, dihydrate

| | C | H | N | O | Cl |
|---|---|---|---|---|---|
| Calc. % | 43,78 | 7,29 | 9,12 | 16,68 | 23,12 |
| Tr. % | 43,97 | 7,32 | 9,03 | 17,09 | 22,80 |

### Exemple 3 : Préparation du tétrachlorhydrate de N,N'-bis-[3-(3,4,5-triméthoxybenzylamino)-propyl]-butane-1,4-diamine

A partir de 5 g de spermine (ou N,N'-bis(3-aminopropyl)-1,4-butane diamine) et selon les mêmes conditions opératoires que celles décrites à l'exemple 1, on obtient 10,40 g de tétrachlorhydrate de N,N'-bis-[3-(3,4,5-triméthoxybenzylamino)-propyl]-butane-1,4-diamine sous forme d'une poudre blanche. Point de fusion : > 250°C (Kofler)
Spectre RMN (500 MHz) dans DMSO : conforme à la structure attendue.
Analyse élémentaire : Produit sous forme tétrachlorhydrate + 1,3 H₂O

| | C | H | N | O | Cl |
|---|---|---|---|---|---|
| Calc. % | 49,22 | 7,74 | 7,66 | 15,97 | 19,41 |
| Tr. % | 49,56 | 7,92 | 7,68 | 16,21 | 19,11 |

### Exemple 4 : Préparation du trichlorhydrate de N-(3,4,5-triméthoxybenzyl)-N',N'-bis-[2-(3,4,5-triméthoxybenzylaminoéthyl]-éthane-1,2-diamine

A partir de 5 g de tris(2-aminoéthyl)amine et selon les mêmes conditions opératoires que celles décrites à l'exemple 1, on obtient 15 g de trichlorhydrate de N-(3,4,5-triméthoxybenzyl)-N',N'-bis[Z-(3,4,5-triméthoxybenzylamino)-éthyl]-éthane-1,2-diamine sous forme d'une poudre blanche.
Spectre RMN (500 MHz) dans DMSO : conforme à la structure attendue.
Analyse élémentaire : Produit sous forme trichlorhydrate + 1,5 H₂O

| | C | H | N | O | Cl |
|---|---|---|---|---|---|
| Calc. % | 52,82 | 7,58 | 6,92 | 20,37 | 13,27 |
| Tr. % | 52,52 | 7,29 | 6,80 | 20,42 | 12,95 |

### Exemple 5 : Préparation de la N,N'-bis-(2-diéthylaminoéthyl)-N,N'-bis-(3,4,5-triméthoxybenzyl)-éthane-1,2-diamine

### (a) Première étape

Dans un tricol de 1 litre, on met en suspension, à température ambiante, 50 g de 3,4,5-triméthoxy benzaldéhyde dans 300 ml de méthanol. On chauffe à 40°C pour obtenir une dissolution complète et on ajoute, goutte à goutte, 8,5 ml d'éthylène diamine. On laisse alors revenir à température ambiante, et le milieu réactionnel, jaune limpide à la fin de l'addition de l'amine, devient rapidement hétérogène avec précipitation d'un solide jaune. On laisse encore sous agitation pendant une heure à température ambiante avant de refroidir à 5°C. Le précipité est collecté par filtration sur verre fritté, lavé abondamment par du méthanol froid (3x150 ml) puis remis en suspension dans 250 ml de méthanol glacé et filtré pour éliminer toute trace d'aldéhyde résiduel. Le précipité blanc est ensuite séché sous vide au dessicateur.

On obtient 49,4 g de diimine attendue de point de fusion=148°C.

### (b) Deuxième étape

Dans un tricol de 1 litre, on met en suspension, à température ambiante, 20 g de la diimine obtenue à la première étape dans 400 ml d'éthanol absolu. On ajoute alors 2,27 g de borohydrure de sodium en pastilles et on chauffe à 55-60°C. On maintient 2h à 55-60°C puis on laisse revenir à température ambiante doucement, et on hydrolyse avec environ 30 ml d'une solution aqueuse d'HCl 6N jusqu'à pH<1. Le milieu réactionnel devient jaune, puis un précipité apparaît rapidement. On refroidit alors à +5°C pendant 1h environ sous faible agitation. Le précipité est alors filtré sur- verre fritté et lavé par 50 ml d'éthanol absolu avant d'être séché sous vide au dessicateur.

On obtient 19,5 g de dichlorhydrate de N,N'-bis-(3,4,5-triméthoxybenzyl) éthylènediamine sous forme de poudre blanche.

### (c) Troisième étape

On dissout 1,9 g de dichlorhydrate de N,N'-bis-(3,4,5-triméthoxybenzyl) éthylènediamine obtenu à l'étape précédente dans 40 ml d'acétone. On ajoute 2,7 g de bromoéthyl diéthylamine en solution dans 40 ml d'acétone, puis 1,40 g de carbonate de potassium, et on porte le milieu réactionnel au reflux pendant 24h.

Après filtration des sels sur papier, les filtrats sont évaporés à sec. Le résidu est repris dans 30 ml de méthanol et 10 ml de méthanol chlorhydrique 2N. Le précipité formé est filtré sur verre fritté, rincé par 10 ml de méthanol, et recristallisé dans 45 ml d'un mélange éther isopropylique-méthanol 4/1. On obtient 1,35 g de N,N'-bis-(2-diéthylaminoéthyl)-N,N'-bis-(3,4,5-triméthoxybenzyl)-éthane-1,2-diamine sous forme d'une poudre blanche.
Point de fusion = 218°C
Spectre RMN (500 MHz) dans DMSO: conforme à la structure attendue
Analyse élémentaire : Produit sous forme tétrachlorhydrate + 0,3 H₂O

| | C | H | N | O | Cl |
|---|---|---|---|---|---|
| Calc. % | 53,03 | 8,14 | 7,28 | 13,10 | 18,46 |
| Tr. % | 53,24 | 8,19 | 7,26 | 13,22 | 18,26 |

### EXEMPLES DE COMPOSITION COSMETIQUE

### Exemple A

On prépare une émulsion, selon les techniques classiques, en procédant au mélange des ingrédients suivants :
- Composé de l'exemple 2 0,1 %
- PEG 50 oxyéthyléné 3 %
- Stéarate de mono/diglycéryle 3 %
- Huile de vaseline 24 %
- Alcool cétylique 5 %
- Eau q.s.p. 100 %

### Exemple B

On prépare une émulsion, selon les techniques classiques, en procédant au mélange des ingrédients suivants :
- Composé de l'exemple 1 0,02 %
- Palmitate d'octyle 10 %
- Isostéarate de glycéryle 4 %
- Huile de vaseline 24 %
- Vitamine E 1 %
- Glycérol 3 %
- Eau q.s.p. 100 %

### Exemple C

On prépare la composition suivante, selon les techniques classiques, en procédant au mélange des ingrédients suivants :
- Composé de l'exemple 2 0,02 %
- Huile de jojoba 13 %
- p-hydroxybenzoate d'alkyle (Alkyl parabens) 0,05 %
- Sorbate de potassium 0,3 %
- Cyclopentadiméthylsiloxane 10 %
- Alcool stéarylique 1 %
- Acide stéarique 4 %
- Stéarate de polyéthylène glycol 3 %
- Vitamine E 1 %
- Glycérol 3 %
- Eau q.s.p. 100 %

### EXEMPLES DE COMPOSITION PHARMACEUTIQUE

### Exemple D : Suspension buvable

- Composé de l'exemple 1 0,10 g
- Ethanol à 90% 1,00 g
- Sorbitol à 70% 0,50 g
- Saccharinate de sodium 0,01 g
- p-hydroxybenzoate de méthyle 0,04 g
- Arôme qs
- Eau 5 ml

### Exemple E : Comprimé

- Composé de l'exemple 1 0,10 g
- Amidon 0,12 g
- Phosphate bicalcique 0,20 g
- Lactose 0,06 g
- Stéarate de magnésium 0,02 g

## Revendications

1. Dérivés benzyle(s) substitué(s) de polyalkylène-polyamines correspondant à la formule suivante : 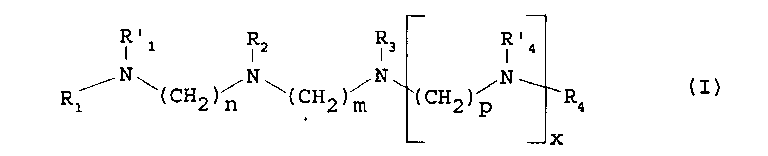 dans laquelle :
n et m = 2, 3 ou 4,
p = 2 ou 3,
x = 0, 1 ou 2,
R₁ et R₄, identiques ou différents, représentent
(i) un radical alkyle, linéaire ou ramifié, en C₁-C₄, ou
(ii) un radical de formule : 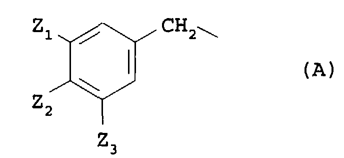
dans laquelle :
Z₁ représente OH ou OR,
Z₂ et Z₃ représentent H, OH ou OR, R étant un radical alkyle, linéaire ou ramifié, en C₁-C₈,
R₂ et R₃, identiques ou différents, représentent H, un radical alkyle, linéaire ou ramifié, en C₁-C₄, un radical de formule (A) ou un radical de formule (B) suivante : 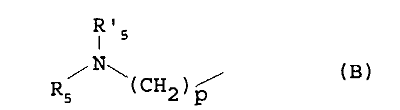 R'₁, R'₄ et R'₅ représentent H ou un radical alkyle, linéaire ou ramifié, en C₁-C₄, et R₅ représente un radical alkyle, linéaire ou ramifié, en C₁-C₄ ou un radical de formule (A),
sous réserve que l'un au moins des radicaux R₁, R₂, R₃, R₄ ou R₅ représente un radical de formule (A), et que lorsque R₁ et R₄, identiques, représentent un radical de formule (A), x étant 0 ou 1, Z₁ ou Z₁ et Z₂, identiques, ne peuvent représenter OR, R étant un radical alkyle en C₁-C₆,
et leurs sels et complexes métalliques.

2. Composés selon la revendication 1, caractérisés par le fait que le radical alkyle, linéaire ou ramifié en C₁-C₄ est choisi parmi les radicaux méthyle, éthyle, isopropyle et tert-butyle.

3. Composés selon la revendication 1, caractérisés par le fait que le radical alkyle, linéaire ou ramifié en C₁-C₈ est choisi parmi les radicaux méthyle, éthyle, isopropyle, tert-butyle, pentyle, hexyle, iso-hexyle, et octyle.

4. Composés selon la revendication 1, caractérisés par le fait que l'un au moins des radicaux R₁, R₂, R₃, R₄ et/ou R₅ représente un radical de formule (A) dans laquelle Z₁, Z₂ et Z₃, identiques ou différents, représentent OH ou OR, R étant un alkyle, linéaire ou ramifié, en C₁-C₈.

5. Composés selon l'une quelconque des revendications précédentes, caractérisés par le fait que les radicaux Z₁, Z₂ et Z₃ sont identiques et représentent un groupe méthoxy.

6. Composés selon l'une quelconque des revendications précédentes, caractérisés par le fait que les sels sont des sels d'addition d'un acide minéral choisi parmi l'acide sulfurique, l'acide chlorhydrique, l'acide nitrique et l'acide phosphorique.

7. Composés selon l'une quelconque des revendications 1 à 5, caractérisés par le fait que les complexes sont des complexes formés par addition de chlorure de zinc ou de chlorure de calcium.

8. Composés selon l'une quelconque des revendications précédentes, caractérisés par le fait qu'ils sont choisis parmi :
N-(3,4,5-triméthoxybenzyl)-N'{2-[2-(3,4,5-triméthoxybenzylamino)-éthylamino]-éthyl}-éthane-1,2-diamine (tétrachlorhydrate),
N-(3,4,5-triméthoxybenzyl)-N'(2-{2-[2-(3,4,5-triméthoxybenzylamino)-éthylamino]-éthylamino}-éthyl)-éthane-1,2-diamine (pentachlorhydrate),
N,N'-bis-[3-(3,4,5-triméthoxybenzylamino)-propyl]-butane-1,4-diamine-(tétrachlorhydrate),
N,N'-bis-(2-diéthylaminoéthyl)-N,N'-bis-(3,4,5-triméthoxybenzyl)-éthane-1,2-diamine,
N-(3,4,5-triméthoxybenzyl)-N',N'-bis[2-(3,4,5-triméthoxybenzylamino)-éthyl]éthane-1,2-diamine.

9. Composition cosmétique ou pharmaceutique, caractérisée par le fait qu'elle contient dans un véhicule cosmétiquement ou pharmaceutiquement acceptable au moins un composé de formule (I) tel que revendiqué dans l'une quelconque des revendications 1 à 8.

10. Composition selon la revendication 9, caractérisée par le fait qu'elle contient le composé de formule (I) en une proportion de 0,001 à 10 % en poids par rapport au poids total de la composition.

11. Composition selon la revendication 9 ou 10, caractérisée par le fait qu'elle contient en outre au moins une substance active choisie parmi les antilipoperoxydants, les réducteurs biologiques, les désactivateurs d'oxygène singulet, les enzymes, une superoxyde dismutase (SOD), le complexe Mn-desferal ou le di-isopropyl salicylate de cuivre, la glutathion peroxydase ou des systèmes à base de sélénium.

12. Utilisation d'un composé de formule (I) selon l'une quelconque des revendications 1 à 8, pour la préparation d'une composition pharmaceutique destinée au traitement de situations de stress oxydant liées à certains états pathologiques.

13. Utilisation d'un composé de formule (I) selon l'une quelconque des revendications 1 à 8 dans la préparation d'une composition cosmétique et/ou pharmaceutique destinée à prévenir le vieillissement cutané.

14. Utilisation d'un composé de formule (I) selon l'une quelconque des revendications 1 à 8 en tant qu'agent antioxydant dans des compositions cosmétiques et pharmaceutiques.

## Patentansprüche

1. Benzylderivat-substituierte Polyalkylenpolyamine der folgenden Formel: 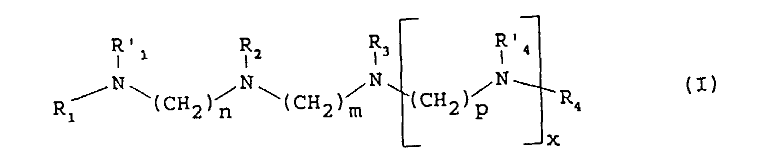 in der:
n und m = 2, 3 oder 4,
p = 2 oder 3,
x = 0, 1 oder 2,
R₁ und R₄ gleich oder verschieden sind und bedeuten:
(i) einen linearen oder verzweigten C₁-C₄-Alkylrest, oder
(ii) einen Rest der Formel: 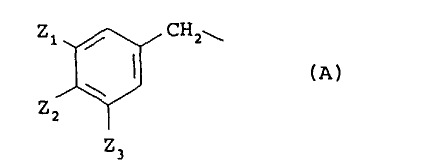
in der:
Z₁ OH oder OR bedeutet,
Z₂ und Z₃ H, OH oder OR bedeuten, R ein linearer oder verzweigter C₁-C₈-Alkylrest ist,
R₂ und R₃ gleich oder verschieden sind und H, einen linearen oder verzweigten C₁-C₄-Alkylrest, einen Rest der Formel (A) oder einen Rest der folgenden Formel (B) bedeuten: 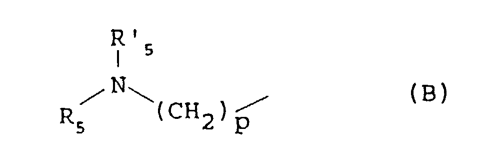
R'₁, R'₄ und R'₅ H oder einen linearen oder verzweigten C₁-C₄-Alkylrest bedeuten, und
R₅ einen linearen oder verzweigten C₁-C₄-Alkylrest oder einen Rest der Formel (A) bedeutet,
mit der Maßgabe, daß mindestens einer der Reste R₁, R₂, R₃, R₄ oder R₅ einen Rest der Formel (A) bedeutet, und wenn R₁ und R₄ identisch sind und einen Rest der Formel (A) bedeuten, x 0 oder 1 ist, Z₁ oder Z₁ und Z₂ identisch sind, nicht OR bedeuten können, wobei R ein C₁-C₆-Alkylrest ist,
und ihre Salze und Metallkomplexe.

2. Verbindung gemäß Anspruch 1, dadurch gekennzeichnet, daß der lineare oder verzweigte C₁-C₄-Alkylrest unter Methyl-, Ethyl-, Isopropyl- und tert.-Butylresten ausgewählt ist.

3. Verbindung gemäß Anspruch 1, dadurch gekennzeichnet, daß der lineare oder verzweigte C₁-C₈-Alkylrest unter den Methyl-, Ethyl-, Isopropyl-, tert.-Butyl-, Pentyl-, Hexyl-, Isohexyl- und Octylresten ausgewählt ist.

4. Verbindung gemäß Anspruch 1, dadurch gekennzeichnet, daß mindestens einer der Reste R₁, R₂, R₃, R₄, und/oder R₅ einen Rest der Formel (A) bedeutet, wobei Z₁, Z₂ und Z₃, die gleich oder verschieden sind, OH oder OR bedeuten, wobei R ein linearer oder verzweigter C₁-C₈-Alkylrest ist.

5. Verbindung nach einem der vorstehenden Ansprüche, dadurch gekennzeichnet, daß die Reste Z₁, Z₂ und Z₃ gleich sind und eine Methoxygruppe bedeuten.

6. Verbindung nach einem der vorstehenden Ansprüche, dadurch gekennzeichnet, daß die Salze Additionssalze einer Mineralsäure sind, ausgewählt aus Schwefelsäure, Salzsäure, Salpetersäure und Phosphorsäure.

7. Verbindung nach einem der Ansprüche 1 bis 5, dadurch gekennzeichnet, daß die Komplexe solche darstellen, die durch Addition von Zinkchlorid oder Calciumchlorid gebildet sind.

8. Verbindungen nach einem der vorstehenden Ansprüche, dadurch gekennzeichnet, daß sie ausgewählt sind aus:
N-(3,4,5-Trimethoxybenzyl)-N'-{2-[2-(3,4,5-trimethoxybenzylamino)-ethylamino]-ethyl}-ethan-1,2-diamin(tetrachlorhydrat),
N-(3,4,5-Trimethoxybenzyl)-N'-(2-{2-[2-(3,4,5-trimethoxybenzylamino)-ethylamino]-ethylamino}-ethyl)-ethan-1,2-diamin (pentachlorhydrat),
N,N'-Bis-[3-(3,4,5-trimethoxybenzylamino)-propyl]-butan-1,4-diamin (tetrachlorhydrat),
N,N'-Bis-(2-diethylaminoethyl)-N,N'-bis-(3,4,5-trimethoxybenzyl)-ethan-1,2-diamin,
N-(3,4,5-Trimethoxybenzyl)-N',N'-bis-[2-(3,4,5-trimethoxybenzylamino)-ethyl]-ethan-1,2-diamin.

9. Kosmetische oder pharmazeutische Zubereitung, dadurch gekennzeichnet, daß sie in einem kosmetisch oder pharmazeutisch annehmbaren Träger mindestens eine Verbindung der Formel (I) gemäß einem der Ansprüche 1 bis 8 enthält.

10. Zubereitung gemäß Anspruch 9, dadurch gekennzeichnet, daß sie die Verbindung der Formel (I) in einem Verhältnis von 0,001 bis 10 Gew.-%, bezogen auf das Gesamtgewicht der Zubereitung, enthält.

11. Zubereitung nach Anspruch 9 oder 10, dadurch gekennzeichnet, daß sie außerdem mindestens eine aktive Substanz, ausgewählt aus Antilipoperoxidantien, biologischen Reduktionsmitteln, Singulett-Sauerstoffdesaktivatoren, Enzymen einer Superoxiddismutase (SOD), dem Mn-Desferalkomplex oder Kupferisopropylsalicylat, der Glutathionperoxidase oder Systemen auf Basis von Selen enthält.

12. Verwendung einer Verbindung der Formel (I) gemäß einem der Ansprüche 1 bis 8 für die Herstellung einer pharmazeutischen Zubereitung, bestimmt zur Behandlung von Situationen oxidativen Stresses, die mit bestimmten pathologischen Zuständen verbunden sind.

13. Verwendung einer Verbindung der Formel (I) nach einem der Ansprüche 1 bis 8 für die Herstellung einer kosmetischen und/oder pharmazeutischen Zubereitung, bestimmt zur Prävention des Alterns von Haut.

14. Verwendung einer Verbindung der Formel (I) gemäß einem der Ansprüche 1 bis 8 als Antioxidans in kosmetischen oder pharmazeutischen Zubereitungen.

## Claims

1. Substituted benzyl derivatives of polyalkylene polyamines corresponding to the following formula: 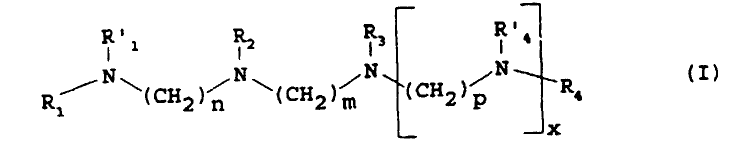 in which:
n and m = 2, 3 or 4,
p = 2 or 3,
x = 0, 1 or 2,
R₁ and R₄, which may be identical or different, represent
(i) a linear or branched C₁-C₄ alkyl radical or
(ii) a radical of formula: 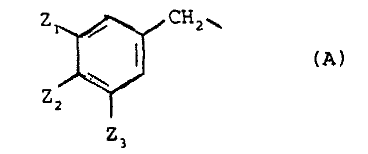
in which:
Z₁ represents OH or OR,
Z₂ and Z₃ represent H, OH or OR, R being a linear or branched C₁-C₈ alkyl radical,
R₂ and R₃, which may be identical or different, represent H, a linear or branched C₁-C₄ alkyl radical, a radical of formula (A) or a radical of formula (B) below: 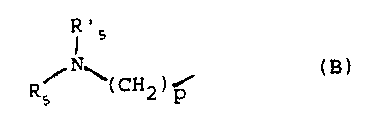
R'₁, R'₄ and R'₅ represent H or a linear or branched C₁-C₄ alkyl radical, and
R₅ represents a linear or branched C₁-C₄ alkyl radical or a radical of formula (A),
with the proviso that at least one of the radicals R₁, R₂, R₃, R₄ or R₅ represents a radical of formula (A), and that when R₁ and R₄, which are identical, represent a radical of formula (A), x being 0 or 1, Z₁ or Z₁ and Z₂, which are identical, cannot represent OR, R being a C₁-C₆ alkyl radical,
and their salts and metal complexes.

2. Compounds according to Claim 1, characterized in that the linear or branched C₁-C₄ alkyl radical is chosen from the methyl, ethyl, isopropyl and tert-butyl radicals.

3. Compounds according to Claim 1, characterized in that the linear or branched C₁-C₈ alkyl radical is chosen from the methyl, ethyl, isopropyl, tert-butyl, pentyl, hexyl, isohexyl and octyl radicals.

4. Compounds according to Claim 1, characterized in that at least one of the radicals R₁, R₂, R₃, R₄ and/or R₅ represents a radical of formula (A) in which Z₁, Z₂ and Z₃, which are identical or different, represent OH or OR, R being a linear or branched C₁-C₈ alkyl.

5. Compounds according to any one of the preceding claims, characterized in that the radicals Z₁, Z₂ and Z₃ are identical and represent a methoxy group.

6. Compounds according to any one of the preceding claims, characterized in that the salts are addition salts of an inorganic acid chosen from sulphuric acid, hydrochloric acid, nitric acid and phosphoric acid.

7. Compounds according to any one of Claims 1 to 5, characterized in that the complexes are complexes formed by addition of zinc chloride or of calcium chloride.

8. Compounds according to any one of the preceding claims, characterized in that they are chosen from:
N-(3,4,5-trimethoxybenzyl)-N-{2-[2-(3,4,5-trimethoxybenzylamino)ethylamino]ethyl}ethane-1,2-diamine (tetrahydrochloride),N-(3,4,5-trimethoxybenzyl)-N'(2-{2-[2-(3,4,5-trimethoxybenzylamino)ethylamino]ethylamino}ethyl)ethane-1,2-diamine (pentahydrochloride),
N,N'-bis[3-(3,4,5-trimethoxybenzylamino)propyl]butane-1,4-diamine (tetrahydrochloride),
N,N'-bis(2-diethylaminoethyl)-N,N'-bis(3,4,5-trimethoxybenzyl)ethane-1,2-diamine,
N-(3,4,5-trimethoxybenzyl)-N',N'-bis[2-(3,4,5-trimethoxybenzylamino)ethyl]ethane-1,2-diamine.

9. Cosmetic or pharmaceutical composition, characterized in that it contains, in a cosmetically or pharmaceutically acceptable vehicle, at least one compound of formula (I) as claimed in any one of Claims 1 to 8.

10. Composition according to Claim 9, characterized in that it contains the compound of formula (I) in a proportion of from 0.001 to 10% by weight relative to the total weight of the composition.

11. Composition according to Claim 9 or 10, characterized in that it also contains at least one active substance chosen from antilipoperoxidizing agents, biological reducing agents, singlet-oxygen deactivators, enzymes, a superoxide dismutase (SOD), the Mn-desferal complex or copper diisopropyl salicylate, glutathione peroxidase or selenium-based systems.

12. Use of a compound of formula (I) according to any one of Claims 1 to 8, for the preparation of a pharmaceutical composition intended for the treatment of oxidative stress conditions associated with certain pathological states.

13. Use of a compound of formula (I) according to any one of Claims 1 to 8, in the preparation of a cosmetic and/or pharmaceutical composition intended to prevent ageing of the skin.

14. Use of a compound of formula (I) according to any one of Claims 1 to 8 as an antioxidant in cosmetic and pharmaceutical compositions.
